Europäisches Patentamt

(19) European Patent Office    (11) Publication number: **0 043 856**

Office européen des brevets                                    **B1**

(12)                    **EUROPEAN PATENT SPECIFICATION**

(45) Date of publication of patent specification: **05.12.84**    (51) Int. Cl.³: **A 61 B 5/10**

(21) Application number: **81900701.4**

(22) Date of filing: **22.01.81**

(86) International application number:
**PCT/US81/00086**

(87) International publication number:
**WO 81/02098 06.08.81 Gazette 81/19**

(54) **FLEXIBLE CALIBRATOR.**

(30) Priority: **24.01.80 US 114979**

(43) Date of publication of application:
**20.01.82 Bulletin 82/03**

(45) Publication of the grant of the patent:
**05.12.84 Bulletin 84/49**

(84) Designated Contracting States:
**AT CH DE FR GB LI LU NL SE**

(56) References cited:
**FR-A- 516 301**
**US-A-1 638 766**
**US-A-1 858 997**
**US-A-2 221 138**
**US-A-2 244 735**
**US-A-2 887 703**
**US-A-3 661 148**
**US-A-3 867 945**
**US-A-3 938 504**
**US-A-3 973 556**
**US-A-4 020 829**

(73) Proprietor: **FOGARTY, Thomas J.**
**770 Welch Road Suite 201**
**Palo Alto, CA 94304 (US)**

(72) Inventor: **FOGARTY, Thomas J.**
**770 Welch Road Suite 201**
**Palo Alto, CA 94304 (US)**

(74) Representative: **Schmitz, Jean-Marie et al**
**Office Dennemeyer S.à.r.l. 21-25 Allée Scheffer**
**P.O. Box 41**
**L-2010 Luxembourg (LU)**

Courier Press, Leamington Spa, England.

## Description

The present invention concerns a device for measuring the degree of stenosis of arterial vessel comprising a flexible normally straight tubular shaft and a flexible normally straight stiffener member slidable within said shaft and the invention relates more particularly to a flexible calibrator which is used to measure the diameter of the lumen in a stenotic segment of blood vessel when the vessel proximal to the stenosis is highly tortuous. At its distal end the calibrator is provided with a bulb-shaped or oval member which is used as a gauge member to determine the lumen size of the vessel.

The prior art known consists of the presently used calibrator instruments. They are provided with lumen-sizing ovals but are rigidly constructed. Their rigidity prevents them from being advanced through sharply winding segments of a vessel in order to reach and measure the desired area of stenosis.

The FR—A—1 504 917 discloses a catheter comprising an insert in the shape of a guide wire which is fully positioned in place, following which the catheter is guided into place by the wire.

In US—A—4 020 829 an insert is also shown, selectively slidable within a catheter to control the shape of the distal end of the catheter. The catheter has a normally curved distal end and the insert has a normally straight end, or vice-versa.

A principal object of the invention is to provide a calibrator with a flexible shaft which enables the calibrator oval to be advanced through tortuous segments of a vessel in order to reach a given area of stenosis. Said calibrator has an elongated carrier having variable stiffness and flexibility characteristics.

The flexible calibrator of the present invention is characterized in comprising a lumen-sizing member and a closed rounded tip member at the distal end of said shaft and said flexible stiffener member being slidable relative to said shaft to vary the degree of flexibility of the distal portion of said shaft.

In order that the invention may be fully understood, reference is made to the accompanying drawings.

Figure 1 is a view in perspective of the flexible calibrator of the invention.

Figure 2 shows the calibrator initially positioned for passage through a tortuous segment to a stenotic segment of a vessel.

Figure 3 is a similar view showing the instrument traversing the tortuous segment of the vessel.

Figure 4 is a similar view showing the calibrator oval of the instrument in sizing disposition to the stenotic segment of the vessel.

Figure 5 is a view in elevation and in section of the preferred embodiment of the subject calibrator.

Figure 6 is a broken-away view in elevation of a further embodiment of the subject calibrator.

A vessel 10 is provided with a proximal incision 12, an intermediate tortuous segment 14, and a distal stenotic segment 16 having a lumen 18 which is to be sized by use of the calibrator of the present invention comprising a handle 20, a calibrator oval 22 and a shaft or carrier 24 interconnecting the handle and oval. The shaft comprises a flexible sheath 26 formed of wound wire 28. The ends of the coiled wire 28 are suitably attached, as by soldering and cementing, to a rounded tip member 30 and to handle member 32. The carrier 24 further comprises a relatively stiff but flexible wire stiffener 34 for the flexible sheath 26. The stiffener wire is attached to handle member 36. Handle members 32 and 36 are adapted to be connected together as by threads 38 and 40. When the handle members 32 and 36 are connected together the distal end of wire 34 fits within socket 42 of tip member 30. The oval member 22 is formed of a soft, pliable material such as silicone.

The manner of use of the calibrator is shown in Figs. 2—4. In Fig. 2, the instrument has been introduced into the vessel 10 through the incision 12 and moved along the vessel to a point adjacent the tortuous segment 14. The wire 34 occupies the full length of the shaft or carrier 24. The stiffness of the shaft 24 is such that further movement of the instrument is resisted by the proximal end of the tortuous segment 14. The operator therefore backs the handle member 36 off of handle member 32 to withdraw enough of the length of the wire stiffener 34 from the flexible sheath 26 to enable the distal, unstiffened portion of sheath 26 to pass through the tortuous segment 14 as the instrument is fed along the vessel by movement of the handle member 32 toward incision 12. The oval 22 becomes finally positioned in the lumen 18 of stenotic segment 16. It typically requires the use of several such instruments, each with a calibration oval of a slightly different size, to correctly calibrate lumen 18.

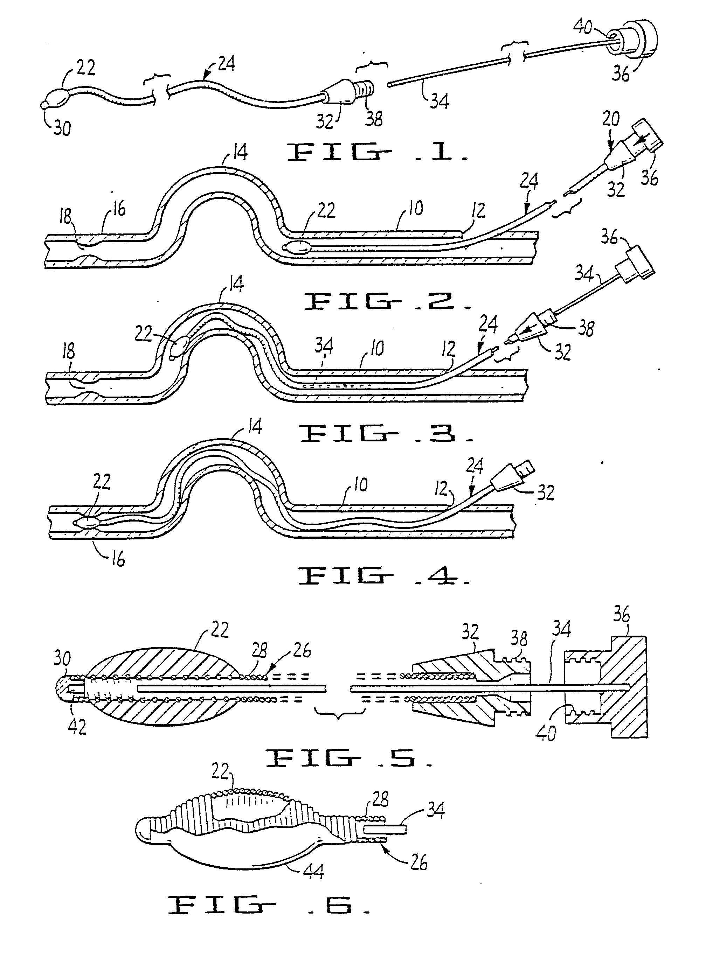

The calibrator may be used with the wire stiffener 34 fully in place for the calibration of the lumen of a stenotic segment of a non-tortuous vessel. It may be used, as above described, when the vessel proximal to the stenosis is highly tortuous, sufficient length of the stiffener or stylet 34 being withdrawn from the shaft 24 in order to position the pliable calibrator oval 22 as shown in Fig. 4.

A preferred construction of the instrument is shown in Fig. 5. The turns of wire 28 are wound tightly upon each other throughout the length of sheath 26 except for that portion of the sheath which is contained within the oval 22. Have the turns of wire are spaced apart from each other such that the silicone material seeps into the lumen of the guide wire body during molding process, thereby insuring that the oval 22 will not slip off of the guide wire during use of the calibrator.

A further embodiment of the instrument is shown in Fig. 6. Here the core of the oval 22 is formed of tightly wound turns of larger diameter of wire 28. The wire core of oval 22 is then covered with a thin plastic or silicone skin 44. The corrugated surface of the wire core of the oval serves as a good anchor for the skin 44.

## Claims

1. A device for measuring the degree of stenosis of arterial vessel comprising a flexible normally straight tubular shaft (24) and a flexible normally straight stiffener member (34) slidable within said shaft, characterized in comprising a lumen-sizing member (22) and a closed rounded tip member (30) at the distal end of said shaft (24) and said flexible stiffener member (34) being slidable relative to said shaft (24) to vary the degree of flexibility of the distal portion of said shaft (24).

2. The device according to claim 1, characterized in further comprising handle means at the proximal end of said shaft (24) comprising a pair of interfitting members (32, 36) one of said members (32) being attached to said shaft (24) and the other of said members (36) being attached to said stiffener member (34).

3. The device according to claim 1, characterized by said flexible shaft (24) comprising a helically wound spring wire member (28), said lumen-sizing member (22) being formed of a molded material which extends between turns of said wire member (28).

4. The device according to claim 3, characterized by the turns of said wire member (28) being normally in abutting relation with each other but being in spaced relation with each other within said lumen-sizing member (22).

5. The device of claim 1, characterized by said flexible shaft (24) comprising a helically wound spring wire member (28), said lumen-sizing member (22) being generally oval in shape and being formed by said helically wound spring wire member (28), and a flexible, resilient coating of plastic material over said lumen-sizing member (22).

## Revendications

1. Un dispositif pour mesurer le degré de sténose d'un vaisseau artériel comprenant un arbre (24) tubulaire normalement droit flexible et un élément (34) raidisseur normalement droit flexible glissant dans cet arbre, caractérisé en ce qu'il comprend un élément (22) pour mesurer la dimension 20 de la lumière et un élément de tête (30) arrondi, fermé à l'éctrémité distale de cet arbre (24) et cet élément raidisseur flexible (34) pouvant glisser par rapport à cet arbre (24) pour varier le degré de flexibilité de la partie distale de cet arbre (24).

2. Appareil selon la revendication 1, caractérisé en ce qu'il comprend en outre une poignée à l'extrémité proximale de cet arbre (24) comprenant une paire d'éléments ajustés (22, 26) l'un de ces éléments (32) étant fixé à cet arbre (24) et l'autre de ces éléments (36) étant attaché à cet élément raidisseur (34).

3. Appareil selon la revendication 1, caractérisé en ce que cet arbre flexible (24) comprend un élément de ressort en fil (28) enroulé hélicoïdalement, cet élément de mesure de la lumière (22) étant formé d'une matière de moulage qui s'étend entre les tours de cet élément en fil (28).

4. Appareil selon la revendication 3, caractérisé en ce que les tours de cet élément en fil (28) sont disposés normalement l'un contre l'autre mais sont écartés l'un de l'autre dans cet élément de mesure de la lumière (22).

5. Appareil selon la revendication 1, caractérisé en ce que cet arbre flexible (24) comprend un élément de ressort en fil (28) enroulé hélicoïdalement, cet élément de mesure de la lumière (22) étant de forme généralement ovale et étant constitué par cet élément de ressort en fil (28) enroulé hélicoïdalement, et un revêtement flexible élastique de matière plastique au-dessus de cet élément de mesure de la lumière (22).

## Patentansprüche

1. Vorrichtung zum Messen des Grades der Verengung von arteriellen Gefässen, welche einen biegsamen, normalerweise geraden rohrförmigen Schaft (24) und ein biegsames, normalerweise gerades Versteifungsteil (34), das in dem Schaft gleitbar ist, umfasst, dadurch gekennzeichnet, dass sie ein Hohlraummessteil (22) und ein geschlossenes abgerundetes Spitzenteil (30) an dem fernen Ende des Schaftes (24) aufweist und das flexible Versteifungsteil (34) relativ zu dem Schaft (24) gleitbar ist, um den Grad der Biegsamkeit des fernen Teiles des Schaftes (24) zu verändern.

2. Vorrichtung nach Anspruch 1, dadurch gekennzeichnet, dass sie ferner eine Griffeinrichtung an dem nahen Ende des Schaftes (24) aufweist, die ein Paar von ineinanderpassenden Teilen (32, 36) umfasst, wobei eines der Teile (32) an dem Schaft (24) befestigt und das andere Teil (36) an dem Versteifungsteil (34) befestigt ist.

3. Vorrichtung nach Anspruch 1, dadurch gekennzeichnet dass der biegsame Schaft (24) ein schraubenförmig gewickeltes Federdrahtteil (28) aufweist, wobei das Hohlraummessteil (22) aus einem geformten Material hergestellt ist, das sich zwischen den Windungen des Drahtteiles (28) erstreckt.

4. Vorrichtung nach Anspruch 3, dadurch gekennzeichnet, dass die Windungen des Drahtteiles (28) normalerweise in Anlage aber innerhalb des Hohlraummessteiles (22) voneinander beabstandet sind.

5. Vorrichtung nach Anspruch 1, dadurch gekennzeichnet dass der biegsame Schaft (24)

ein schraubenförmig gewickeltes Federdrahtteil (28) aufweist, wobei das Hohlraummessteil (22) eine im allgemeinen ovale Form hat und durch das schraubenförmig gewickelte Federdrahtteil (28) und einer biegsamen elastischen Schicht aus Kunststoff auf dem Hohlraum-messteil (22) gebildet wird.

FIG .1.

FIG .2.

FIG .3.

FIG .4.

FIG .5.

FIG .6.